Europäisches Patentamt

European Patent Office

Office européen des brevets

(11) Numéro de publication: **0 346 553 B1**

## FASCICULE DE BREVET EUROPEEN

(45) Date de publication de fascicule du brevet: **26.08.92**  (51) Int. Cl.5: **A61L 2/20**, //G02C13/00

(21) Numéro de dépôt: **88440047.4**

(22) Date de dépôt: **14.06.88**

(54) **Procédé de décontamination et de nettoyage de prothèses oculaires, en particulier de lentilles de contact, et dispositif pour la mise en oeuvre de ce procédé.**

(43) Date de publication de la demande:
**20.12.89 Bulletin 89/51**

(45) Mention de la délivrance du brevet:
**26.08.92 Bulletin 92/35**

(84) Etats contractants désignés:
**AT BE CH DE ES GB GR IT LI LU NL SE**

(56) Documents cités:
**EP-A- 0 170 602**
**DE-A- 3 524 407**

(73) Titulaire: **Nold, Yves**
**9, rue du Parc**
**F-57100 Thionville(FR)**

(72) Inventeur: **Nold, Yves**
**9, rue du Parc**
**F-57100 Thionville(FR)**

(74) Mandataire: **Nuss, Pierre**
**10, rue Jacques Kablé**
**F-67000 Strasbourg(FR)**

## Description

La présente invention concerne le domaine des dispositifs de correction de la vue, notamment de l'entretien de tels dispositifs, en particulier de l'entretien des prothèses oculaires, et a pour objet un procédé de décontamination et de nettoyage de telles prothèses oculaires, en particulier de lentilles de contact.

L'invention a également pour objet un dispositif pour la mise en oeuvre de ce procédé.

Le port de lentilles de contact pose, pour l'utilisateur de telles lentilles, un problème de protection contre l'infection microbienne des yeux, de sorte qu'il associe obligatoirement un autre problème, à savoir celui concernant le produit d'entretien desdites lentilles. En effet, un tel produit d'entretien requiert, outre une simplicité d'utilisation, des qualités d'efficacité microbiologique et des propriétés nettoyantes.

Il existe actuellement plusieurs procédés de traitement des lentilles de contact qui peuvent être regroupés selon leur mode d'action en deux catégories, à savoir chimique et physique, qui présentent, toutefois, un certain nombre d'inconvénients.

Les procédés chimiques mettent en oeuvre des solutions isotoniques et stérilisantes qui contiennent des conservateurs tels que le gluconate de chlorhéxidine, des dérivés mercuriels ou des ammoniums quaternaires. L'emploi de ces solutions exige, cependant, de nombreuses opérations contraignantes pour l'utilisateur et, en outre, ces solutions sont la cause de réactions de toxicité et d'irritation oculaires relativement nombreuses.

Il a également été proposé de traiter les lentilles avec une solution à trois pour cent d'eau oxygénée. Un tel procédé est efficace du point de vue de la destruction des germes, mais nécessite la neutralisation de la solution par un agent réducteur afin d'éviter de provoquer des brûlures aux yeux de l'utilisateur.

EP-A-170 602 décrit un procédé de decontamination de prothèses oculaires caractérisé en ce qu'il utilise de l'ozone produit par ionisation dans un tube ionisant. Cet ozone migre à travers une membrane filtrante et diffuse ensuite dans une solution isotonique de trempage des lentilles.

Parmi les procédés physiques, on peut citer, notamment, l'ébullition, qui est un procédé confirmé pour la stérilisation de l'eau mais qui présente, cependant, l'inconvénient de provoquer une lente opacification des lentilles de contact due à la dénaturation du matériel muco-protéique.

Les procédés connus actuellement ne permettent donc pas de traiter, de manière simple, et sans effets secondaires indésirables, des lentilles de contact.

La présente invention a pour but de pallier ces inconvénients.

Elle a, en effet, pour objet, un procédé de décontamination et de nettoyage de prothèses oculaires, en particulier de lentilles de contact, caractérisé en ce qu il consiste essentiellement à traiter lesdites lentilles de contact dans une solution isotonique de trempage, disposée dans un récipient, par barbotage d'ozone produit par ionisation dans un tube ionisant et migrant au moyen d'une pompe et d'un conduit de liaison du tube ionisant vers le récipient de réception de la solution isotonique et des lentilles, ce barbotage étant réalisé pendant une durée prédéterminée, de préférence comprise entre trente minutes et deux heures.

L'invention a également pour objet un dispositif pour la mise en oeuvre de ce procédé, caractérisé en ce qu'il est essentiellement constitué par un boîtier renfermant un tube ionisant, dont une extrémité est reliée à une pompe à air, et dont l'autre extrémité est reliée à un conduit de transport de l'ozone produit, ce conduit étant pourvu d'un capillaire, par un ensemble électrique de commande du tube ionisant et de la pompe à air, et par un récipient amovible de réception d'une solution isotonique et des lentilles à traiter, dans lequel pénètre le capillaire du conduit de transport.

L'invention sera mieux comprise grâce à la description ci-après, qui se rapporte à un mode de réalisation préféré, donné à titre d'exemple non limitatif, et expliqué avec référence aux dessins schématiques annexés, dans lesquels :

la figure 1 est une vue en perspective du dispositif conforme à l'invention ;

la figure 2 est une vue en perspective, par l'avant, du dispositif suivant la figure 1, le couvercle du boîtier étant enlevé ;

la figure 3 est une vue analogue à celle de la figure 2, mais en perspective par l'arrière ;

la figure 4 est un schéma du circuit électrique de commande, et

la figure 5 est une vue analogue à celle de la figure 2 d'une variante de réalisation de l'invention.

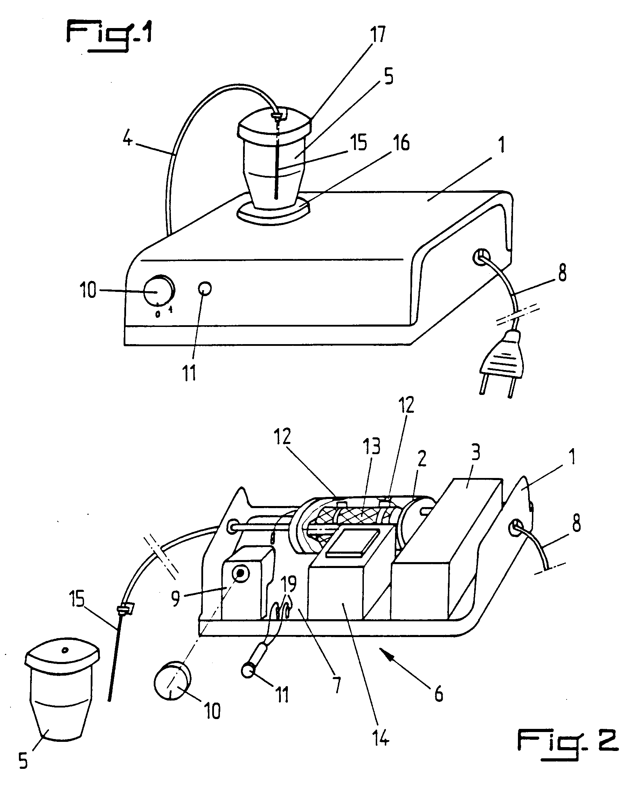

Conformément à l'invention, et comme le montrent plus particulièrement, à titre d'exemple, les figures 1 à 3 des dessins annexés, le dispositif pour la décontamination et le nettoyage de prothèses oculaires, en particulier de lentilles de contact est essentiellement constitué par un boîtier 1, dans lequel est monté un tube ionisant 2, dont une extrémité est reliée à une pompe à air 3, et dont l'autre extrémité est reliée à un conduit 4 de transport de l'ozone produit, pourvu d'un capillaire 15, et par un récipient amovible 5 de réception d'une solution isotonique et des lentilles à traiter, dans lequel pénètre le capillaire 15 du conduit 4. Le tube ionisant 2 et la pompe à air 3 sont commandés par un ensemble électrique 6.

L'ensemble électrique 6 est constitué par un circuit imprimé 7 sur lequel est raccordé un cordon d'alimentation 8, une minuterie 9, mécanique, électronique, ou autre, pourvue d'un bouton de mise en marche 10 et d'un voyant 11 témoin de fonctionnement,ainsi que la pompe à air 3 et le tube ionisant 2.

Le tube ionisant 2, qui est fixé sur le circuit imprimé 7 au moyen de colliers 12, renferme une lampe à ozone 13 alimentée par un transformateur 14. Ainsi, lors de la mise en marche du dispositif au moyen du bouton 10 et de la minuterie 9, la pompe à air 3 souffle de l'air neuf dans le tube ionisant 2, dans lequel ledit air 9 est transformé en ozone au moyen de la lampe à ozone 13, puis l'air chargé d'ozone est pulsé à travers le conduit 4 et le capillaire 15 dans le récipient 5. Le récipient amovible 5 de réception d'une solution isotonique et des lentilles à traiter est monté sur le boîtier 1, ou contre ce dernier, au moyen d'un disque de positionnement 16, et son couvercle de fermeture 17 est pourvu d'un perçage d'introduction du capillaire 15.

La minuterie 9 commandée par le bouton 2 est destinée à couper l'alimentation électrique du dispositif après une durée prédéterminée et est reliée au circuit imprimé 7 au moyen de deux cosses 18, le voyant 11 témoin de fonctionnement étant également relié au circuit imprimé au moyen de deux cosses 19. L'ozone utilisé dans le procédé conforme à l'invention est un agent chimique l'état gazeux, qui, dans des conditions normales de pression et de température, est constitué de trois atomes d'oxygène et qui s'autodétruit en tendant à se dissocier en permanence d'après la transformation :

$$O_3 \rightarrow O_2 + O \ .$$

Les atomes d'oxygène O s'unissent deux à deux pour former de l'oxygène $O_2$. Ainsi, l'ozone produit dans le tube ionisant 2 disparaît très rapidement lorsque la source qui le génère (lampe à ozone 13) est arrêtée, et il ne peut se produire d'effet secondaire lié à son absorption et à sa décharge ultérieures dans les yeux de l'utilisateur.

Le but du procédé conforme à l'invention, est de réaliser la dissolution de l'ozone dans une solution isotonique de conservation des lentilles de contact, tout en isolant mécaniquement le milieu liquide contenant lesdites lentilles du tube ionisant 2 qui travaille en atmosphère sèche.

Selon une caractéristique de l'invention, la concentration d'ozone dissout dans la solution isotonique est avantageusement comprise entre 0,1 mg/l et 10 mg/l, et est, de préférence, de 2,5 mg/l. Grâce à une telle concentration, le dispositif conforme à l'invention permet d'assurer une préservation de toute contamination microbiologique après le traitement.

Le fonctionnement du dispositif conforme à l'invention est décrit ci-après, à titre d'exemple, à propos d'une opération de nettoyage et de décontamination de lentilles. Après fixation du récipient 5 sur le disque de positionnement 16, le capillaire 15 du conduit 4 est introduit à travers le couvercle 17 dudit récipient 5, et l'ensemble électrique 6 est raccordé au circuit électrique au moyen de son cordon d'alimentation 8. Puis le dispositif est mis en marche par actionnement du bouton 10 de la minuterie 9 et le voyant 11 s'allume. La lampe à ozone 13 du tube ionisant 2 est alimentée par le courant issu du transformateur 14 et produit ainsi de l'ozone qui est pulsé par la pompe 3 à travers le conduit 4 et son capillaire 15 pénétrant dans le récipient 5. Du fait de la propulsion de l'ozone au moyen de la pompe 3, il se produit un barbotage dans le récipient 5, de sorte qu'il s'exerce une action mécanique sur les lentilles,ce qui a pour effet un décollement des dépôts.

Après une durée de fonctionnement prédéterminée, par exemple de l'ordre de deux heures, préréglée au moyen de la minuterie 9, l'opération de nettoyage et de décontamination est terminée et l'arrêt du dispositif s'effectue automatiquement.

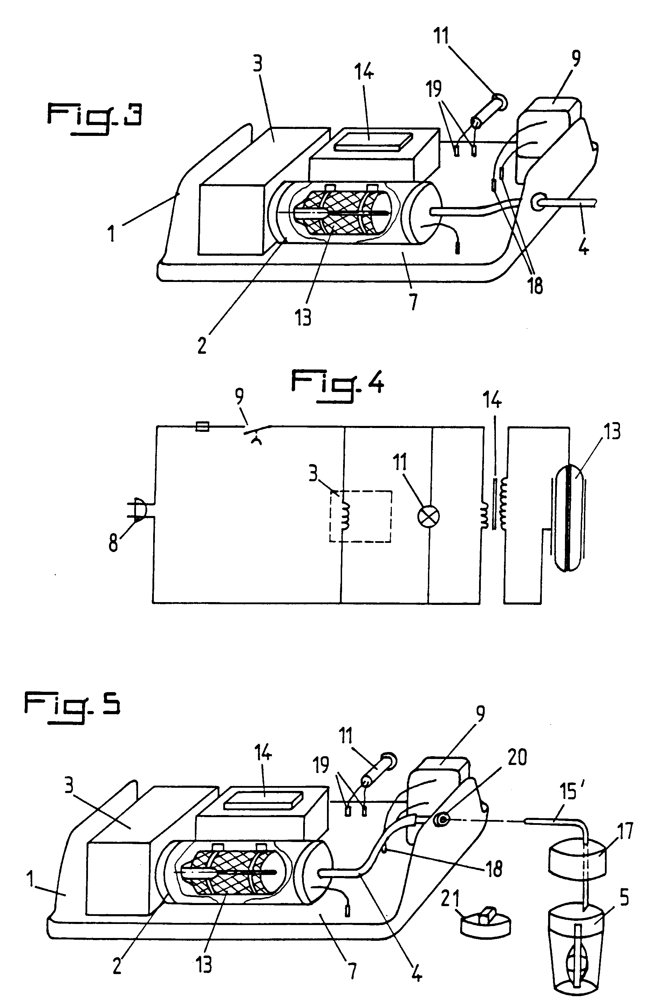

La figure 5 représente une variante de réalisation de l'invention, dans laquelle le conduit 4 de transport de l'ozone produit, raccordé à une extrémité, au tube ionisant 2, est relié à son autre extrémité à un embout 20 débouchant sur la paroi du boîtier 1, et le couvercle de fermeture 17 du récipient 5 est muni d'un capillaire 15' dont l'extrémité externe est coudée et pénètre à glissement sans jeu dans l'embout 20 du conduit 4. Grâce à ce mode de réalisation, il est possible de disposer le récipient 5 directement contre le boîtier 1 et d'insérer l'extrémité libre du capillaire 15' dans l'embout 20 pour le traitement, le récipient 5 avec son bouchon 17 et le capillaire 15' pouvant être séparés du boîtier 1 en position de non-utilisation, de sorte que, dans cette position, le boîtier 1 se présente sans aucun élément en saillie sur ses parois. En outre, le récipient 5 peut avantageusement être muni d'un bouchon hermétique 21 pour le transport.

L'expérimentation a montré qu'une durée de traitement de deux heures était suffisante pour un nettoyage et une décontamination efficaces.

Cette expérimentation a porté sur l'étude de l'activité bactéricide de souches microbiennes et de souches sauvages de germes isolées des conjonctives ou des lentilles de sujets équipés de lentilles de contact.

L'étude de l'activité bactérienne a porté sur cinq souches microbiennes, à savoir :
- Staphylococcus aureus 209P
- Streptococcus faecalis ATCC 10541
- Escherichia coli ATCC 10536
- Pseudomonas aeruginosa CNCM A22
- Candida albicans.

A cet effet, des suspensions en soluté de chlorure de sodium à 9 p. 1000 titrant $10^5$ à $10^6$ germes par ml (suspension A) ont été préparées à partir de cultures de 24 heures sur gélose Trypcase-soja ou Malt-agar (candida).

Protocole expérimental :

Le récipient 5 du dispositif est rempli avec 5 ml de suspension microbienne A fraîchement préparée. Le barbotage de l'ozone est enclenché au moyen de la minuterie 9 qui maintient le fonctionnement du dispositif pendant deux heures a température du laboratoire.

A la fin du cycle ainsi programmé, la suspension est diluée de $10^{-1}$ à $10^{-4}$ en soluté stérile de chlorure de sodium à 9 p. 1000. Ensuite, 1 ml de chaque dilution obtenue, ainsi que de la suspension mère est incorporé en "gélose standard pour dénombrement" (ou en "Malt-agar" pour candida) en boîte de Pétri, à raison de deux boîtes par dilution.

Parallèlement, on dilue et on incorpore de la même manière une fraction de la suspension A non traitée par l'ozone, afin de déterminer la concentration initiale en micro-organismes vivants (témoin), et les boîtes sont placées 37° C ou 30° C (candida) pendant au moins 48 heures avant de procéder au dénombrement des colonies.

Les résultats figurant dans le tableau ci-après montrent une excellente activité bactérienne à l'égard de quatre souches bactériennes et de la souche de levure testées. En effet, le nombre de survivants a été réduit d'au moins quatre puissances de 10 et même de plus de cinq dans la plupart des cas avec les bactéries, à l'issue des deux heures de traitement par l'ozone au moyen du dispositif conforme à l'invention.

| Souche | Essai n° | Nb de bactéries (oulevurees)/ml témoin | Survivants |
|---|---|---|---|
| S. aureus 209P | 1 | $0,25 \times 10^6$ | 0 |
| E. Coli ATTCC 10536 | 2 | $1,26 \times 10^6$ | 0 |
| | 3 | $1,00 \times 10^6$ | 0 |
| S. faecalis ATTCC 10541 | 4 | $2,16 \times 10^6$ | 0 |
| | 5 | $0,53 \times 10^6$ | 0 |
| P. aeruginosa CNCM A 22 | 6 | $0,55 \times 10^6$ | 0 |
| | 7 | $0,55 \times 10^5$ | 0 |
| C. albicans | 8 | $0,82 \times 10^5$ | 0* |
| | 9 | $0,60 \times 10^5$ | 0* |

* 3 survivants

Le dispositif conforme à l'invention a montré une excellente efficacité microbicide, aussi bien sur les quatre souches bactériennes examinées que sur la souche de levure Candida Albicans, dans les conditions normales de fonctionnement.

Par ailleurs, des lentilles ont été ensemencées de souches sauvages de germes isolés choisies et connues pour provoquer des infections oculaires graves ou pour attaquer la lentille (Céphalosporium), à savoir, six bactéries :
- Staphylococcus aureus
- Acinetobacter calcoaceticus variété anitratus
- Pseudomonas aéruginosa
- Klebsiella pneumoniae
- Enterobacter agglomerans
- Serratia marcescens,

EP 0 346 553 B1

et sur deux champignons :
- Candida albicans
- Cephalosporium sp.

Pour les bactéries, la culture a été effectuée, pendant 24 à 48 heures à 37°C en atmosphère de $CO_2$ à 10 % sur un milieu gélose chocolat enrichie (Pasteur), et pour les champigons à 26°C dans un milieu Sabouraud en boîte de Pétri, une première lecture étant réalisée au bout de trois jours, et une dernière lecture au bout de sept jours, pour le Céphalosporium.

La suspension de bactéries ou de levures ou de spores a une concentration d'environ $10^5$ ml a été effectué dans le sérum physiologique stérile, et des lentilles hydrophiles à 70 %, ont été immergées dans cette solution, puis placées dans leur support . Ce dernier est ensuite introduit dans un récipient 5 rempli de sérum physiologique contenant la suspension.

Un ensemencement de 50 $\mu$l de la suspension de germes non diluée et des ensemencements des dilutions de 10 en 10 de cette suspension sont pratiqués sur le milieu de culture correspondant et servent de témoin et d'étalon pour la numération éventuelle des germes qui pousseraient dans les tests.

Le dispositif conforme à l'invention est alors mis en route, et à la fin de son cycle de nettoyage on réalise un ensemencement de 50 $\mu$l de sérum physiologique contenant la suspension de germes et un ensemencement des lentilles par passage à la surface du milieu de culture. Les milieux d'ensemencement sont cultivés dans les conditions décrites ci-dessus.

Le tableau ci-après indique le résultat des cultures, après le cycle de désinfection :

| Germes | Suspension | lentille droite | lentille gauche |
|---|---|---|---|
| Staphylococcus a. | Stérile | Stérile | Stérile |
| Acinetobacter c. | Stérile | Stérile | Stérile |
| Pseudomonas a. | Stérile | Stérile | Stérile |
| Klebsiella p. | Stérile | Stérile | Stérile |
| Enterobacter a. | Stérile | Stérile | Stérile |
| Serratia m. | Stérile | Stérile | Stérile |
| Candida a. | Stérile | Stérile | Stérile |
| Cephalosporium sp. | Stérile | Stérile | Stérile |

Comme le montre ce tableau, la décontamination du liquide et des lentilles vis-à-vis des germes testés est complète après le cycle de nettoyage du dispositif, aucun germe survivant n'ayant été mis en évidence.

Par ailleurs, des essais complémentaires de 100 heures de traitement en continu sur différents types de lentilles n'induisent aucune modification du spectre infra-rouge due à une altération du matériau.

Le procédé et le dispositif conformes à l'invention, permettent donc de réaliser le nettoyage et la décontamination de lentilles de contact de manière efficace et sans effet secondaire indésirable. En outre, cinq minutes après l'arrêt du dispositif, l'ozone a disparu dans la solution aqueuse.

La production d'ozone, en fonction du temps de fonctionnement du dispositif, a été dosée au thiosulphate de sodium 0,01 N et est la suivante :

| | |
|---|---|
| 0 à 20 minutes | 552 $\mu$g ozone/5ml |
| 0 à 40 minutes | 872 $\mu$g ozone/5ml |
| 0 à 60 minutes | 1 450 $\mu$g ozone/5ml |
| 0 à 120 minutes(soit 1 cycle complet) | 2 460 $\mu$g ozone/5ml |

Grâce à l'invention, il est possible de réaliser une décontamination et un nettoyage efficaces de prothèses oculaires, en particulier de lentilles de contact, au moyen d'un dispositif de faible encombrement et d'un faible prix de revient.

Bien entendu, l'invention n'est pas limitée au mode de réalisation décrit et représenté aux dessins annexés. Des modifications restent possibles, notamment du point de vue de la constitution des divers éléments, ou par substitution d'équivalents techniques, sans sortir pour autant du domaine de protection de l'invention.

**Revendications**

**1.** Procédé de décontamination et de nettoyage de prothèses oculaires, en particulier de lentilles de contact, caractérisé en ce qu'il consiste essentiellement à traiter lesdites lentilles de contact dans une solution isotonique de trempage, disposée dans un récipient (5), par barbotage d'ozone produit par ionisation dans un tube ionisant (2) et migrant au moyen d'une pompe (3) et d'un conduit (4) de liaison du tube ionisant (2) vers le récipient (5) de réception de la solution isotonique et des lentilles, ce barbotage étant réalisé pendant une durée prédéterminée, de préférence comprise entre trente minutes et deux heures.

2. Procédé, suivant la revendication 1, caractérisé en ce que la concentration d'ozone dissout dans la solution isotonique est avantageusement comprise entre 0,1 mg/l et 10 mg/l, et est, de préférence, de 2,5 mg/l.

3. Dispositif pour la mise en oeuvre du procédé suivant la revendication 1, caractérisé en ce qu'il est essentiellement constitué par un boîtier (1) renfermant un tube ionisant (2),dont une extrémité est reliée à une pompe à air (3), et dont l'autre extrémité est reliée à un conduit (4) de transport de l'ozone produit, ce conduit (4) étant pourvu d'un capillaire (15), par un ensemble électrique (6) de commande du tube ionisant (2) et de la pompe à air (3), et par un récipient amovible (5) de réception d'une solution isotonique et des lentilles à traiter, dans lequel pénètre le capillaire (15) du conduit de transport (4).

4. Dispositif, suivant la revendication 3, caractérisé en ce que l'ensemble électrique (6) est constitué par un circuit imprimé (7) sur lequel est raccordé un cordon d'alimentation (8), une minuterie (9), mécanique, électronique, ou autre, pourvue d'un bouton de mise en marche (10) et d'un voyant (11) témoin de fonctionnement,ainsi que la pompe à air (3) et le tube ionisant (2) .

5. Dispositif, suivant l'une quelconque des revendications 3 et 4, caractérisé en ce que le tube ionisant (2), qui est fixé sur le circuit imprimé (7) au moyen de colliers (12), renferme une lampe à ozone (13) alimentée par un transformateur (14).

6. Dispositif, suivant la revendication 3, caractérisé en ce que le récipient amovible (5) de réception d'une solution isotonique et des lentilles à traiter est monté sur le boîtier (1), ou contre ce dernier, au moyen d'un disque de positionnement (16), et son couvercle de fermeture (17) est pourvu d'un perçage d'introduction du capillaire (15).

7. Dispositif, suivant l'une quelconque des revendications 1 et 6, caractérisé en ce que le conduit (4) de transport de l'ozone produit, est raccordé à une extrémité au tube ionisant (2) et est relié à son autre extrémité à un embout (20) débouchant sur la paroi du boîtier (1), le couvercle de fermeture (17) du récipient (5) étant muni d'un capillaire (15') dont l'extrémité externe est coudée et pénètre à glissement sans jeu dans l'embout (20) du conduit (4).

**Claims**

1. Method of decontaminating and cleaning eye prostheses, in particular contact lenses, characterised in that it essentially involves treating said contact lenses in an isotonic soaking solution arranged in a container (5) by bubbling ozone produced by ionisation in an ionising tube (2) and migrating by means of a pump (3) and a conduit (4) for connecting the ionising tube (2) toward the container (5) for receiving the isotonic solution and the lenses, this bubbling being carried out for a predetermined period of time, preferably of between 30 minutes and 2 hours.

2. Method according to claim 1, characterised in that the concentration of ozone dissolved in the isotonic solution is advantageously between 0.1 mg/l and 10 mg/l and is preferably 2.5 mg/l.

3. Device for carrying out the method according to claim 1, characterised in that it essentially consists of a casing (1) containing an ionising tube (2) of which one end is connected to an air pump (3) and of which the other end is connected to a conduit (4) for conveying the ozone produced, this conduit (4) being provided with a capillary (15), of an electrical unit (6) for controlling the ionising tube (2) and the air pump (3), and of a removable container (5) for receiving an isotonic solution and the lenses to be treated, in which the capillary (15) of the conveying conduit (4) penetrates.

4.  Device according to claim 3, characterised in that the electrical unit (6) consists of a printed circuit (7) to which there are connected a supply lead (8), a timer (9) of a mechanical, electronic or other type provided with a start button (10) and an inspection window (11) for monitoring operation, as well as the air pump (3) and the ionising tube (2).

5.  Device according to any one of claims 3 and 4, characterised in that the ionising tube (2) which is fixed on the printed circuit (7) by means of hoops (12) contains an ozone lamp (13) supplied by a transformer (14).

6.  Device according to claim 3, characterised in that the removable container (5) for receiving an isotonic solution and the lenses to be treated is mounted on the casing (1) or against it by means of a positioning disk (16) and its closure cover (17) is provided with a perforation for introduction of the capillary (15).

7.  Device according to any one of claims 1 and 6, characterised in that the conduit (4) for conveying the ozone produced is connected at one end to the ionising tube (2) and is connected at its other end to a joining piece (20) opening at the wall of the casing (1), the closure cover (17) of the container (5) being equipped with a capillary (15') of which the external end is bent and penetrates by sliding without clearance in the joining piece (20) of the conduit (4).

**Patentansprüche**

1.  Verfahren zur Entkeimung und Reinigung von Augenprothesen, insbesondere von Kontaktlinsen, dadurchgekennzeichnet, daß es im wesentlichen darin besteht, die besagten Kontaktlinsen in einer in einem Behälter (5) enthaltenen isotonischen Badelösung durch Durchperlen mit Ozon zu behandeln, wobei das Ozon durch Ionisation in einer Ionisationsröhre (2) erzeugt wird und mittels einer Pumpe (3) und einer Verbindungsleitung (4) von der Ionisationsröhre (2) zu dem die isotonische Lösung und die Kontaktlinsen aufnehmenden Behälter (5) wandert, wobei dieses Durchperlen über eine bestimmte, vorzugsweise zwischen dreißig Minuten und zwei Stunden liegende Dauer durchgeführt wird.

2.  Verfahren nach Anspruch 1, dadurch gekennzeichnet, daß die Konzentration des in der isotonischen Lösung gelösten Ozons vorteilhafterweise zwischen 0,1 mg/l und 10 mg/l, und vorzugsweise bei 2,5 mg/l liegt.

3.  Vorrichtung zur Durchführung des Verfahrens nach Anspruch 1, dadurch gekennzeichnet, daß sie im wesentlichen aus einem die Ionisationsröhre (2) einschließenden Gehäuse (1) besteht, wobei die Ionisationsröhre mit einem Ende an eine Luftpumpe (3) und mit dem anderen Ende an eine Ozonförderleitung (4) angeschlossen ist, wobei diese Leitung (4) mit einem Kapillarröhrchen (15) versehen ist, sowie aus einer elektrischen Steuereinheit (6) der Ionisationsröhre (2) und der Luftpumpe (3), und aus einem abnehmbaren Behälter (5) zur Aufnahme der isotonischen Lösung und der zu behandelnden Kontaktlinsen, in den das Kapillarröhrchen (15) der Förderleitung (4) hineinragt.

4.  Vorrichtung nach Anspruch 3, dadurch gekennzeichnet, daß die elektrische Einheit (6) aus einem gedruckten Schaltkreis (7) besteht, an welchen ein Anschlußkabel (8) angeschlossen ist, eine mechanische, elektronische oder sonstige, mit einem Einschaltknopf (10) und einer Betriebskontrolleuchte (11) versehene Zeitschaltuhr (9), sowie die Luftpumpe (3) und die Ionisationsröhre (2).

5.  Vorrichtung nach einem beliebigen der Ansprüche 3 und 4, dadurch gekennzeichnet, daß die auf dem gedruckten Schaltkreis (7) mittels Schellen (12) befestigte Ionisationsröhre (2) eine von einem Transformator (14) gespeiste Ozonröhre (13) einschließt.

6.  Vorrichtung nach Anspruch 3, dadurch gekennzeichnet, daß der abnehmbare Behälter (5) zur Aufnahme einer isotonischen Lösung und der zu behandelnden Kontaktlinsen mittels einer Positionierungsscheibe (16) auf oder an dem Gehäuse (1) angebracht wird, und daß sein Verschlußdeckel (17) mit einer Bohrung zur Einführung des Kapillarröhrchens (15) versehen ist.

7.  Vorrichtung nach einem beliebigen der Ansprüche 1 und 6, dadurch gekennzeichnet, daß die Förderleitung (4) zum Transport des erzeugten Ozons mit ihrem einen Ende an die Ionisationsröhre (2) und mit ihrem anderen Ende an einen an der Wand des Gehäuses (1) mündenden Stutzen (20) angeschlossen ist, wobei der Verschlußdeckel (17) des Behälters (5) mit einem Kapillarröhrchen (15') versehen ist, dessen äußeres Ende gebogen ist und spielfrei gleitend in den Stutzen (20) der Leitung (4) eindringt.

Fig.1

Fig. 2

9

Fig.3

Fig.4

Fig.5